# EUROPÄISCHE PATENTSCHRIFT

(11) **EP 0 796 608 B1**
(45) Veröffentlichungstag und Bekanntmachung des Hinweises auf die Patenterteilung: **12.03.2003**
(21) Anmeldenummer: 97100908.9
(22) Anmeldetag: 22.01.1997
(51) Int. Cl.: A61K 6/04

(54) **Verwendung einer Gold-Zinnlegierung als Dentallot**
Use of a gold-tin alloy as a solder in dentistry
Utilisation d'un alliage or-étain pour le soudage en art dentaire

(30) Priorität: 24.02.1996 DE 19607104
(43) Veröffentlichungstag der Anmeldung: 24.09.1997
(73) Patentinhaber: Degussa Dental GmbH, 63457 Hanau (DE)
(72) Erfinder: Kempf, Bernd, Dr., 63839 Kleinwallstadt (DE); Schöck, Gernot, 63486 Bruchköbel (DE); Völcker, Alexander, Dr., 63517 Rodenbach (DE)
(74) Vertreter: Stoffregen, Hans-Herbert, Dr. Dipl.-Phys. Patentanwalt

(56) Entgegenhaltungen:
- EP-A- 0 424 775
- EP-A- 0 569 166
- DE-A- 2 636 039
- DE-A- 4 324 738

## Beschreibung

Die Erfindung betrifft die Verwendung einer Gold-Zinnlegierung als Dentallot zum Löten von Teilen aus hochgoldhaltigen Dentallegierungen, die neben Palladium und gegebenenfalls Platin noch Zinn enthalten.

Bei der Herstellung von Zahnersatz müssen in vielen Fällen zum Verbinden von Einzelteilen Lötarbeiten durchgeführt werden. Hierzu benötigt man Lotlegierungen, die ein gutes Benetzungs- und Fließverhalten zeigen, eine ausreichende Härte und Festigkeit aufweisen und vor allem auch mundbeständig sind. Ihre Arbeitstemperatur muß genügend weit unterhalb des Schmelz- bzw. Erweichungspunktes der zu lötenden Dentallegierung liegen, damit es zu keinen Verformungen während des Lötens kommt. Sehr oft enthalten die Lotlegierungen die gleichen Edelmetalle wie die Dentallegierungen, um eine Lokalelementbildung zu unterdrücken. Zur Absenkung des Schmelzbereiches müssen allerdings in den meisten Fällen zusätzliche Unedelmetalle zulegiert werden, um auf eine optimale Arbeitstemperatur zu kommen. Deswegen ist der Gold- bzw. Edelmetallgehalt der Lotlegierungen in der Regel niedriger als der Edelmetallgehalt der zu verbindenden Dentalteile.

Im Zuge eines wachsenden Bewußtseins bezüglich der Biokompatibilität von Zahnersatz ist speziell das Löten in die Kritik geraten, da die Korrosionsanfälligkeit von gelötetem Zahnersatz aufgrund einer elektrisch leitenden Verbindung unterschiedlicher Legierungen und damit möglicher Kontaktkorrosionseffekte zu einer Verschlechterung der Korrosionsbeständigkeit des Zahnersatzes führen kann.

Aus der DE-OS 43 24 738 sind hochgoldhaltige Gold-Palladiumlegierungen bekanntgeworden, die als einziges Unedelmetall 0,7 bis 5,8 Gew.% Zinn enthalten. Diese Legierungen zeigen extrem niedrige Korrosionswerte und sind daher für Dentalanwendungen sehr gut geeignet.

Es war daher Aufgabe der vorliegenden Erfindung, eine Lotlegierung für hochgoldhaltige Gold-Palladium-Legierungen zu finden, die als einziges Unedelmetall Zinn enthält, die alle für ein Lot notwendigen Eigenschaften besitzt und zusätzlich als Legierung als auch im Verbund mit den Dentallegierungen eine sehr hohe Korrosionsbeständigkeit aufweist.

Diese Aufgabe wird erfindungsgemäß durch die Verwendung einer Legierung gelöst, die aus 0,1 bis 2 Gew.% Zinn, 0 bis 5 Gew.% Palladium, 0 bis 5 Gew.% Platin und 0 bis 0,5 Gew.% Iridium, Rhodium und/oder Ruthenium, Rest Gold besteht.

Vorzugsweise enthält die Lotlegierung 0,5 bis 1,5 Gew.% Zinn, 1 bis 3 Gew.% Palladium, 1 bis 3 Gew.% Platin und 0,05 bis 0,4 Gew.% Iridium, Rhodium und/oder Ruthenium, Rest Gold.

Überraschenderweise zeigt es sich, daß diese Lotlegierungen nicht nur als Legierungen keine oder nur sehr geringe Korrosionsraten zeigen, sondern daß sie auch im Lotverbund mit den genannten Dentallegierungen diese überragende Beständigkeit beibehalten.

Diese Lotlegierungen zeichnen sich dadurch aus, daß sie mit Zinn nur ein einziges Nichtedelmetall enthalten, dessen Unbedenklichkeit durch seine vielfältige Verwendung in der Nahrungsmittelindustrie als Zinngeschirr oder als Weißblech belegt ist. Diese Legierungen zeigen ferner ein außergewöhnlich gutes Benetzungsverhalten beim Löten von Dentalaufbrennlegierungen, die in gleicher Weise legierungstechnisch zusammengesetzt sind. Lotverbunde aus diesen Legierungen zeigen so niedrige Korrosionsraten, daß diese bei der Analyse unter der Nachweisgrenze liegen. Sie waren außerdem bereits im Gußzustand, der dem gelöteten Gefügezustand am nächsten kommt, so hohe Härten auf, daß ein mechanisch sehr stabiler Lotverbund entsteht.

In Tabelle 1 sind einige erfindungsgemäße Legierungen zusammengestellt. Neben der Zusammensetzung ist zusätzlich als mechanische Kenngröße die Härte im zahntechnischen Guß sowie im ausgehärteten Zustand und die Arbeitstemperatur (AT) angegeben.

| **Leg.Nr.** | **Au** | **Ir** | **Pt** | **Pd** | **Sn** | **AT** | **HV-G** | **HV-H** |
|---|---|---|---|---|---|---|---|---|
| 1 | 97,6 | 0,2 | 0 | 1 | 1,2 | 1060° | 45 | 38 |
| 2 | 96,6 | 0,2 | 2 | 0 | 1,2 | 1060 | 76 | 99 |
| 3 | 93,9 | 0,2 | 4 | 0 | 1,9 | 1070 | 82 | 145 |
| 4 | 95 | 0,1 | 2 | 2 | 0,9 | 1110 | 108 | 122 |
| HV-G: Härte nach Vickers im Gußzustand HV-H: Härte nach Vickers im ausgehärteten Zustand. | | | | | | | | |

In Tabelle 2 sind die Ergebnisse von Korrosionsuntersuchungen zusammengestellt. Zur Bestimmung der Korrosionsbeständigkeit wurden Korrosionsversuche nach DIN-Entwurf 13927 durchgeführt. Dazu werden Probekörper für 7 Tage in einer 0,1 m Milchsäure- 0,1 m Kochsalzlösung bei 37°C gelagert. Anschließend wird die Korrosionslösung mittels geeigneter Analyseverfahren quantitativ auf die freigesetzten Korrosionsprodukte analysiert.

## Patentansprüche

1. Verwendung einer Gold-Zinnlegierung, bestehend aus 0,1 bis 2 Gew.% Zinn, 0 bis 5 Gew.% Palladium, 0 bis 5 Gew.% Platin und 0 bis 0,5 Gew.% Iridium, Rhodium und/oder Ruthenium, Rest Gold, zum Löten von Dentalteilen aus hochgoldhaltigen Legierungen, die neben Gold nur Palladium, gegebenenfalls Platin und Zinn enthalten.

2. Verwendung einer Gold-Zinnlegierung nach Anspruch 1, bestehend aus 0,5 bis 1,5 Gew.% Zinn, 1 bis 3 Gew.% Palladium, 1 bis 3 Gew.% Platin und 0,05 bis 0,4 Gew.% Iridium, Rhodium und/oder Ruthenium, Rest Gold.

## Claims

1. Use of a gold-tin alloy, comprising 0.1 to 2% by weight tin, 0 to 5% by weight palladium, 0 to 5% by weight platinum and 0 to 5% by weight iridium, rhodium and/or ruthenium, the rest gold, for soldering of dental parts of alloys with a high gold content, that in addition to gold contain only palladium and where necessary platinum and tin.

2. Use of a gold-tin alloy according to claim 1, comprising 0.5 to 1.5% by weight tin, 1 to 3% by weight palladium, 1 to 3% by weight platinum and 0.05 to 0.4% by weight iridium, rhodium and/or ruthenium, the rest gold.

## Revendications

1. Utilisation d'un alliage or-étain composé de 0,1 à 2 % p/p d'étain, de 0 à 5 % p/p de palladium, de 0 à 5 % p/p de platine et de 0 à 0,5 % p/p d'iridium, de rhodium et/ou de ruthénium, le reste étant de l'or, pour le brasage de pièces dentaires en alliages à forte teneur en or contenant outre de l'or uniquement du palladium, et le cas échéant du platine et de l'étain.

2. Utilisation d'un alliage or-étain conformément à la revendication en 1.), composé de 0,5 à 1,5 % p/p d'étain, de 1 à 3 % p/p de palladium, de 1 à 3 % p/p de platine et de 0,05 à 0,4 % p/p d'iridium, de rhodium et/ou de ruthénium, le reste étant de l'or.
